# FASCICULE DE BREVET EUROPEEN

(11) **EP 0 671 156 B1**
(45) Date de publication et mention de la délivrance du brevet: **24.10.2001**
(21) Numéro de dépôt: 95400322.4
(22) Date de dépôt: 15.02.1995
(51) Int. Cl.: A61K 7/00, A61K 9/127

(54) **Procédé de préparation de compositions cosmétiques ou pharmaceutiques comprenant des liposomes**
Verfahren zur Herstellung von Liposomen enthaltenden kosmetischen oder pharmazeutischen Zusammensetzungen
Process to prepare cosmetic or pharmaceutical compositions containing liposomes

(30) Priorité: 16.02.1994 FR 9401755
(43) Date de publication de la demande: 13.09.1995
(73) Titulaire: Aventis Pharma S.A., 92160 Antony (FR)
(72) Inventeur: Dubois, Jean-Luc, F-75019 Paris (FR)

(56) Documents cités:
- EP-A- 0 494 819
- EP-A- 0 560 138
- EP-A- 0 580 459
- DE-C- 4 038 075
- J. STEROID BIOCHEM. MOL. BIOL., vol. 48, no. 1, 1994 pages 55-60, T. BATTMANN ET AL. 'RU 58841, a new specific topical antiandrogen: a candidate of choice for the treatment of acne, androgenetic alopecia and hirsutism.'

## Description

L'invention concerne la préparation de compositions cosmétiques et pharmaceutiques, notamment dermatologiques comprenant des liposomes qui contiennent un composé actif.

Un nombre assez important de préparations cosmétiques ou pharmaceutiques comprenant des principes actifs différents existent actuellement sur le marché. De même des compositions pharmaceutiques et cosmétiques qui contiennent des vésicules du type liposomes sont bien connues, par exemple dans les brevets FR-A 2627385 ou par EP-A 342100.

Le brevet DE 4038075 décrit un procédé d'encapsulation de substances actives lipophiles solides ou liquides pour former des liposomes phospholipidiques contenant de telles substances actives.

La présente invention concerne un procédé de préparation de compositions cosmétiques ou pharmaceutiques comprenant des liposomes qui contiennent un composé actif, caractérisées en ce que les liposomes contiennent comme principe actif au moins un composé de formule (I) : 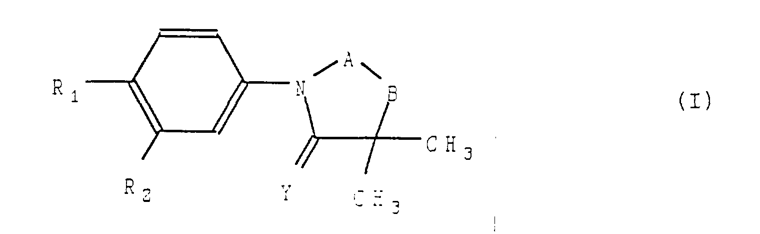 dans laquelle :
R₁ représente un radical cyano ou nitro ou un atome d'halogène,
R₂ représente un radical trifluorométhyle ou un atome d'halogène, le groupement -A-B- est choisi parmi les radicaux 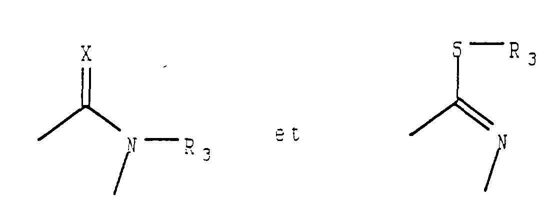 dans lesquels X représente un atome d'oxygène ou de soufre et
R₃ est choisi parmi les radicaux suivants :
   - un atome d'hydrogène,
   - les radicaux alkyle, alkényle, alkynyle, aryle ou arylalkyle ayant au plus 12 atomes de carbone, ces radicaux étant éventuellement substitués par un ou plusieurs substituants choisis parmi les radicaux hydroxy, halogène, mercapto, cyano, acyle ou acyloxy ayant au plus 7 atomes de carbone, aryle, O-aryle, O-aralkyle, S-aryle dans lesquels le radical aryle renfermant au plus 12 atomes de carbone est éventuellement substitué et l'atome de soufre est éventuellement oxydé sous forme de sulfoxyde ou de sulfone, carboxy libre, estérifié, amidifié ou salifié, amino, mono ou dialkylamino ou un radical hétérocyclique comprenant 3 à 6 chaînons et renfermant un ou plusieurs hétéroatomes choisis parmi les atomes de soufre, d'oxygène ou d'azote,
      les radicaux alkyle, alkényle ou alkynyle étant de plus éventuellement interrompus par un ou plusieurs atomes d'oxygène, d'azote ou de soufre éventuellement oxydé sous forme de sulfoxyde ou de sulfone,
      les radicaux aryle et aralkyle étant de plus éventuellement substitués par un atome d'halogène, par un radical alkyle, alkényle ou alkynyle, alkoxy, alkényloxy, alkynyloxy ou trifluorométhyle,
   - les radicaux trialkylsilyle dans lesquels le radical alkyle linéaire ou ramifié comprend au plus 6 atomes de carbone,
   - les radicaux acyle ou acyloxy renfermant au plus 7 atomes de carbone,
Y représente un atome d'oxygène ou de soufre ou un radical =NH, ce procédé étant caractérisé en ce que l'on prépare une émulsion ou suspension aqueuse comprenant un composé actif (par exemple, un composé de formule (I)) et un ou plusieurs composés lipidiques et éventuellement des additifs comme un tampon de pH, un antioxydant ou un antiseptique et que l'on agite l'émulsion ou la suspension à une température comprise entre 40° et 80°C,et que l'on applique ensuite un procédé de réduction de taille des liposomes.

Les composés actifs ont été décrits par exemple dans la demande de brevet européen EP-A 494819. De plus la demande de brevet européen EP-A 0 580 459 présente des composés de ce type.

La présente invention concerne un procédé de préparation de compositions cosmétiques ou pharmaceutiques tel que défini ci-dessus caractérisé en ce que l'on utilise comme composé actif un produit de formule (I) tel que défini ci-dessus, dans laquelle X représente un atome d'oxygène, Y représente un atome d'oxygène, R₂ représente un atome d'halogène ou un radical trifluorométhyle et R₁ représente un radical nitro, un atome d'halogène ou un radical cyano et R₃ représente un atome d'hydrogène ou un radical alkyle ayant au plus 4 atomes de carbone éventuellement substitué par un radical hydroxy ou méthoxy.

La présente invention a pour objet un procédé de préparation de compositions cosmétiques ou pharmaceutiques tel que défini ci-dessus caractérisé en ce que l'on utilise comme composé actif un composé de formule (I') :

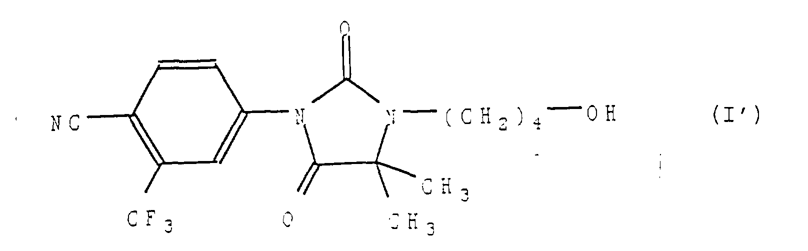

Les définitions utilisées ci-dessus peuvent avoir les valeurs suivantes :

Par alkyle ayant au plus 12 atomes de carbone on entend par exemple les valeurs méthyle, éthyle, propyle, isopropyle, butyle, isobutyle, sec-butyle, tert-butyle, pentyle, isopentyle, sec-pentyle, tert-pentyle, néo-pentyle, hexyle, isohexyle, sec-hexyle, tert-hexyle, heptyle, octyle, décyle, undécyle, dodécyle, linéaires ou ramifiés.

On préfère les radicaux alkyle ayant au plus 6 atomes de carbone et notamment les radicaux méthyle, éthyle, propyle, isopropyle, n-butyle, isobutyle, terbutyle, pentyle linéaire ou ramifié, hexyle linéaire ou ramifié.

Par alkényle ayant au plus 12 et préférentiellement 4 atomes de carbone on entend par exemple les valeurs suivantes :
vinyle, allyle, 1-propényle, butényle, pentényle, hexenyle.

Parmi les valeurs alkényle, on préfère les valeurs allyle ou butényle.

Par alkynyle ayant au plus 12 et préférentiellement 4 atomes de carbone on entend par exemple les valeurs suivantes :
éthynyle, propargyle, butynyle, pentynyle ou hexynyle.

Parmi les valeurs alkynyle, on préfère la valeur propargyle.

Par aryle on entend les radicaux aryles carbocycliques tels que le phényle ou le naphtyle ou les aryles hétérocycliques à 5 ou 6 chaînons comportant un ou plusieurs hétéroatomes choisis de préférence parmi l'oxygène, le soufre et l'azote. Parmi les aryles hétérocycliques à 5 chaînons on peut citer les radicaux furyle, thiényle, pyrrolyle, thiazolyle, oxazolyle, imidazolyle, thiadiazolyle, pyrazolyle, isoxazolyle.

Parmi les aryles hétérocycliques à 6 chaînons on peut citer les radicaux pyridyle, pyrimidinyle, pyridazinyle, pyrazinyle.

Parmi les radicaux aryles condensés on peut citer les radicaux indolyle, benzofurannyle, benzothiényle, quinoléïnyle.

On préfère le radical phényle.

Par arylalkyle on entend les radicaux résultant de la combinaison des radicaux alkyle cités précédemment éventuellement substitués et les radicaux aryle également cités ci-dessus, éventuellement substitués.

On préfère les radicaux benzyle, phényléthyle ou triphénylméthyle.

Par halogène, on entend bien entendu, les atomes de fluor, de chlore, de brome ou d'iode.

On préfère les atomes de fluor, de chlore ou de brome.

Comme exemples particuliers de radicaux alkyle substitués par un ou plusieurs halogènes, on peut citer les monofluoro, chloro, bromo ou iodométhyle, les difluoro, dichloro ou dibromométhyle, le trifluorométhyle.

Comme exemples particuliers de radicaux aryles ou aralkyles substitués, on peut citer ceux dans lesquels le radical phényle est substitué, par un atome de fluor ou par un radical méthoxy ou trifluorométhyle.

Par radical acyle, on entend de préférence un radical ayant au plus 7 atomes de carbone tel que le radical acétyle, propionyle, butyryle ou benzoyle, mais il peut également représenter un radical valéryle, hexanoyle, acryloyle, crotonoyle ou carbamoyle : on peut également citer le radical formyle.

Par radical acyloxy, on entend les radicaux dans lesquels les radicaux acyle ont la signification indiquée ci-dessus et par exemple les radicaux acétoxy ou propionyloxy.

Par carboxy estérifié on entend par exemple les radicaux tels que les radicaux alkyloxycarbonyle par exemple méthoxycarbonyle, éthoxycarbonyle, propoxycarbonyle, butyl ou tertbutyloxycarbonyle, cyclobutyloxycarbonyle, cyclopentyloxycarbonyle ou cyclohexyloxycarbonyle.

On peut également citer des radicaux formés avec les restes esters facilement clivables tels que les radicaux méthoxyméthyle, éthoxyméthyle ; les radicaux acyloxyalkyle tels que pivaloyloxyméthyle, pivaloyloxyéthyle, acétoxyméthyle ou acétoxyéthyle ; les radicaux alkyloxycarbonyloxy alkyle tels que les radicaux méthoxycarbonyloxy méthyle ou éthyle, les radicaux isopropyloxycarbonyloxy méthyle ou éthyle.

Une liste de tels radicaux esters peut-être trouvée par exemple dans le brevet européen EP 0 034 536.

Par carboxy amidifié on entend les radicaux du type 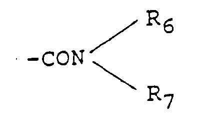 dans lesquels les radicaux R₆ et R₇ identiques ou différents représentent un atome d'hydrogène ou un radical alkyle ayant de 1 à 4 atomes de carbone tels que les radicaux méthyle, éthyle, propyle, isopropyle, butyle, isobutyle, secbutyle ou tert-butyle.

Parmi les radicaux 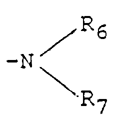 on préfère les radicaux amino, mono ou diméthylamino.

Le radical 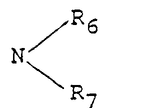 peut également représenter un hétérocycle qui peut ou non comporter un hétéroatome supplémentaire. On peut citer les radicaux pyrrolyle, imidazolyle, pyridyle, pyrazinyle, pyrimidyle, indolyle, pipéridino, morpholino, pipérazinyle. On préfère les radicaux pipéridino ou morpholino.

Par carboxy salifié on entend les sels formés par exemple avec un équivalent de sodium, de potassium, de lithium, de calcium, de magnésium ou d'ammonium. On peut également citer les sels formés avec les bases organiques telles que la méthylamine, la propylamine, la triméthylamine, la diéthylamine, la triéthylamine.

On préfère le sel de sodium.

Par radical alkylamino on entend les radicaux méthylamino, éthylamino, propylamino ou butyl, linéaire ou ramifié, amino. On préfère les radicaux alkyle ayant au plus 4 atomes de carbone, les radicaux alkyle peuvent être choisis parmi les radicaux alkyle cités ci-dessus.

Par radical dialkylamino on entend par exemple les radicaux diméthylamino, diéthylamino, méthyléthylamino. Comme précédemment on préfère les radicaux alkyle ayant au plus 4 atomes de carbone choisis dans la liste indiquée ci-dessus.

Par radical hétérocyclique renfermant un ou plusieurs hétéroatomes, on entend par exemple les radicaux monocycliques, hétérocycliques saturés tels que les radicaux oxirannyle, oxolannyle, dioxolannyle, imidazolidinyle, pyrazolidinyle, pipéridyle, pipérazinyle ou morpholinyle.

Par radicaux alkyle, alkényle, ou alkynyle éventuellement interrompus par un hétéroatome choisis parmi les atomes de soufre, d'oxygène ou d'azote, on entend les radicaux comprenant dans leur structure un ou plusieurs de ces atomes, identiques ou différents, ces hétéroatomes ne pouvant évidemment pas être situés à l'extrémité du radical. On peut citer par exemple les radicaux alkoxyalkyle tels que méthoxyméthyle, méthoxyéthyle, méthoxypropyle et méthoxybutyle ou encore les radicaux alkoxy alkoxyalkyle tels que méthoxyéthoxyméthyle.

Par radical trialkylsilyle dans lequel le radical alkyle comprend au plus 6 atomes de carbone, on entend par exemple, les radicaux triméthylsilyle, triéthysilyle, (1,1-diméthyléthyl) diméthylsilyle.

Lorsque les produits de formule (I) comportent un radical amino salifiable par un acide il est bien entendu que ces sels d'acides peuvent également être utilisés. On peut citer les sels formés avec les acides chlorhydrique ou méthanesulfonique, par exemple.

Le procédé de préparation des produits de formule générale (I) telle que définie ci-dessus est décrit par EP-A 494819.

Les compositions cosmétiques ou pharmaceutiques telles que préparées dans la présente invention contiennent avantageusement de 0,001 à environ 20 % en poids, de préférence de 0,01 à 10 % en poids du ou des composés actifs de formule (I) par rapport au poids de la composition totale.

Les compositions comprenant des liposomes telles que préparées dans la présente invention peuvent se présenter sous différentes formes, par exemple sous forme de gel, de crème, de lait, de baume ou de lotion.

Le principe actif est au moins en partie incorporé dans des vésicules de type liposomes, notamment à une proportion de plus de 20 %, préférablement à une proportion de plus de 50 %, et spécialement plus de 90 %.

En principe, les compositions contenant des liposomes peuvent être préparées selon la technique suivante, qui a été décrite dans FR-A 2627385 :
Les principes actifs sont tout d'abord préparés par exemple, sous forme d'une solution aqueuse, s'ils sont solubles dans l'eau.
Les vésicules des liposomes sont constituées d'une phase lipidique comprenant au moins une des substances suivantes :
   - les phospholipides, d'origine naturelle ou synthétique,
   - les phospholipides associés à des glycérides,
   - les phospholipides associés à des glycolipides,
   - les cérébrosides,
   - les sphingolipides,
   - les céphalines,
   - les phosphoaminolipides,
   - les cérébroglucosides,
   - les gangliosides,
   éventuellement combinés à du cholestérol, naturel ou synthétique.

Cette phase lipidique est dissoute dans un solvant volatil, variant selon le type de substance choisie, par exemple un solvant organique tel que le chloroforme ou le méthanol.

La solution lipidique obtenue est placée dans un ballon puis évaporée sous pression réduite dans un évaporateur rotatif, jusqu'à formation d'un film sur les parois du ballon.

Puis on ajoute, sous agitation constante, la solution aqueuse des principes actifs à encapsuler, et obtient ainsi une suspension qui est ensuite soumise à des ultrasons.

On obtient ainsi une suspension de vésicules type liposome incorporant au moins en partie les principes actifs en solution aqueuse.

L'encapsulation des principes actifs ainsi réalisée entraîne une optimisation de leur activité cosmétodynamique.

L'application topique d'un principe actif encapsulé dans des liposomes permet de concentrer le principe actif dans les glandes sébacées, d'obtenir une concentration plus élevée et plus durable dans l'épiderme et dans le derme in vivo, que celle obtenue par les applications topiques classiques, et enfin de limiter l'effet systémique en minimisant le passage du principe actif dans la circulation sanguine ; les effets secondaires des produits sont de ce fait, atténués.

La pénétration percutanée du principe actif dépend donc du composé actif et du mode de fabrication des liposomes.

La préparation des compositions contenant des liposomes utilisant la phase lipidique dissoute dans un solvant volatil a des désavantages parce qu'on peut toujours retrouver des traces de solvant dans la composition finale.

L'invention a également pour objet une méthode de préparation des liposomes qui n'utilise pas de solvants organiques et qui produit des résultats satisfaisants pour ce qui concerne la pénétration percutanée du principe actif.

L'invention concerne donc un procédé de préparation d'une composition pharmaceutique ou cosmétique comprenant des liposomes, caractérisé en ce que l'on prépare une émulsion ou suspension aqueuse comprenant un composé actif (par exemple, un composé de formule (I)) et un ou plusieurs composés lipidiques et éventuellement des additifs comme un tampon de pH, un antioxydant ou un antiseptique et que l'on agite l'émulsion ou la suspension à une température comprise entre 40° et 80°C, préférablement entre 40° et 75°C, spécialement entre 60° et 70°C, et que l'on applique ensuite un procédé de réduction de taille des liposomes. Préférentiellement, on prépare à chaud une dispersion solide comprenant le composé actif, un ou plusieurs composés lipidiques et éventuellement des additifs et on dispense cette dispersion solide dans de l'eau. Ce procédé est réalisé sans utiliser de solvants organiques.

L'invention a donc particulièrement pour objet un procédé tel que défini ci-dessus caractérisé en ce que l'on prépare tout d'abord un mélange des constituants lipidiques comprenant un ou plusieurs principes actifs, les phospholipides et les conservateurs, que l'on chauffe ensuite jusqu'à la fusion à une température comprise entre 40 et °80°C, puis en ce que l'on met en présence sous agitation le mélange ainsi obtenu, les composés hydrophiles comprenant le tampon de pH, un ou plusieurs antiseptiques pour obtenir la solution de liposomes que l'on soumet à un procédé de réduction de taille.

L'originalité de l'invention résulte aussi en ce que le principe actif a un bas point de fusion ≃ 100°C et que ce principe actif est dissous ou soluble dans le phospholipide ou le mélange de phospholipides à basse température (par exemple entre 50° et 70°C). De plus cette basse température minimise l'oxydation ou la dégradation des phospholipides et correspond à la température optimale d'émulsification des phospholipides dans de l'eau ou dans du tampon de pH.

Le procédé selon l'invention peut être réalisé avec différents principes actifs mais préférablement, on utilise comme composé actif, un composé de formule (I) telle que définie ci-dessus et tout particulièrement le composé de formule (I') :

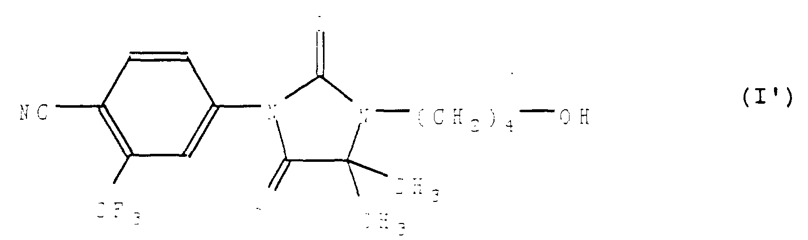

On obtient des compositions très intéressantes en appliquant la méthode décrite.

Comme composé lipidique, on utilise préférablement un phospholipide naturel, semi-synthétique ou synthétique, par exemple le Lipoïd E 100.35, le Lipoïd EPC 3 ou le Lipoïd SPC 3 hydrogéné ou non (qui sont commercialisés par la Société Diététique Française de Formulation et de Fabrication, 24, Avenue Hoche, 75008 Paris, France). Les trois produits ont un poids moléculaire entre 777 et 790, une température de transition entre 45° et 60°C et consistent pour plus de 90 % de phosphatidylcholine d'oeuf ou de phosphatidylcholine de soja.

Comme tampon de pH, on peut par exemple utiliser un tampon phosphate (pH 7).

Comme anti-oxidant l'acétate d'α-tocophérol ou l'α-tocophérol peut être employé (par exemple à une concentration comprise entre 0,1 et 5 %, préférablement entre 0,5 et 2 % du poids des composés lipidiques).

Comme antiseptique, on utilise des composés connus en général comme par exemple les parahydroxybenzoates de propyle, d'éthyle ou de méthyle à une concentration de 0,01 à 0,5 % et de préférence entre 0,05 et 0,1 %, ou encore le Bronopol à la concentration de 0,1 %.

Pour la préparation de médicaments, on peut aussi ajouter aux compositions décrites, d'autres additifs. Par exemple pour la préparation du gel, la carboxyméthylcellulose (Blanose®) ou un autre gélifiant utilisé classiquement est ajouté lentement à la suspension liposomiale sous agitation à température ambiante. Des gels à 0,5 %, 0,75 %, 1 % et 1,25 % de Blanose® ont été réalisés.

Selon le procédé décrit, on applique normalement un rapport molaire phospholipide/principe actif entre 1,0 et 20,0 mais préférablement entre 5,5 et 15,0, spécialement entre 8 et 12 pour le composé de formule (I') comme principe actif.

Le pourcentage d'encapsulation du composé actif croît normalement avec le rapport molaire lipide/composé actif.

La concentration en principe actif dans la suspension aqueuse est dépendante de la concentration en phospholipides. Normalement, on utilise une concentration de plus de 10 mmol de phospholipides par litre de phase aqueuse, préférablement entre 30 et 400 mmol/l, spécialement entre 60 et 300 mmol/l.

En principe, les diverses méthodes de réduction de taille pour les liposomes sont applicables. Préférentiellement, on utilise un microfluidiseur ® (voir S. Vemuri et al., "Large-scale Production of Liposomes by a Micro-fluidizer", Drug Develop. Ind. Pharm. ; 1990 ; 16,15 ; 2243-2256). Dans la chambre d'intéraction du microfluidiseur, deux flux de la suspension de liposomes entrent en collision sous forte pression et grande vitesse. Il en résulte une réduction de la taille des vésicules.

La température lors de la fusion, de l'émulsification et du passage dans le microfluidiseur normalement doit être supérieure à la température de transition des composés lipides. Préférentiellement, le procédé de préparation selon l'invention est caractérisé en ce que l'on agite l'émulsion ou la suspension à une température comprise entre 40° et 75°C.

Les liposomes obtenus par l'utilisation du microfluidiseur ont des tailles identiques ou inférieures à ceux obtenus par les méthodes utilisant les ultrasons (voir E. Mayhew et al., "A practical method for the large scale manufacture of liposomes", Pharmaceutical Manufacturing, 1985, 8, 18-22).

L'émulsion ou la suspension comprenant les liposomes peut passer dans le microfluidiseur une ou plusieurs fois, normalement on applique 1 à 6, préférablement 2 à 5 passages au microfluidiseur, car ensuite on n'observe plus de changements importants de la taille des liposomes.

Le procédé de préparation selon l'invention est donc caractérisé en ce que l'on utilise comme procédé de réduction de taille des liposomes un ou plusieurs passages de l'émulsion ou de la suspension dans un microfluidiseur.

Le procédé de préparation de la composition cosmétique ou pharmaceutique est bien reproductible et peut être réalisé à l'échelle industrielle. De plus, on peut obtenir des préparations avec des teneurs en phospholipides et principe actif élevées.

### EXEMPLE 1 : préparation du principe actif : 4-(4,4-diméthyl 2,5-dioxo 3-(4-hydroxy butyl) 1-imidazolidinyl) 2-(trifluorométhyl) benzonitrile

### a) Condensation

A une suspension de 104 mg d'hydrure de sodium dans 0,8 cm³ de diméthylformamide, on ajoute 600 mg de 4-(4,4-diméthyl-2,5-dioxo-1-imidazolidinyl)-2-(trifluorométhyl)-benzonitrile obtenu comme à l'exemple 8 de la demande de brevet européen n° 0 494 819 dans 5 cm³ de diméthylformamide en maintenant la température inférieure à 20°C. Après 10 minutes d'agitation, on ajoute 445 mg de 4-chloro t-butyl diméthylsilyléther et 300 mg d'iodure de sodium. On chauffe 16 heures à 50°C, refroidit à température ambiante, ajoute 87 mg d'hydrure de sodium puis de nouveau 400 mg de l'éther chloré et 267 mg d'hydrure de sodium, et chauffe 1 heure supplémentaire. On ramène à température ambiante, verse sur 60 cm³ d'eau contenant 600 mg de phosphate monopotassique. On extrait à l'éther, lave la phase organique à l'eau, sèche et évapore le solvant. On chromatographie le résidu sur silice (éluant chlorure de méthylène-acétone (99-1)), recueille 526 mg de produit utilisé tel quel pour le stade suivant ;

### b) Clivage.

On mélange le produit obtenu ci-dessus dans 5 cm³ de méthanol et 1,5 cm³ d'acide chlorhydrique 2N. On agite 40 minutes à température ambiante, verse sur 30 cm³ d'eau, extrait au chlorure de méthylène, lave la phase organique à l'eau, sèche et évapore le solvant. Après chromatographie du résidu sur silice (éluant chlorure de méthylène-acétone (9-1) on récupère les fractions Rf = 0,15 et après recristallisation dans l'éther isopropylique, on obtient 307 mg de produit attendu. F = 102-103°C.

Préparation du 4-chloro t-butyl diméthylsilyléther utilisé au départ de l'exemple.

On mélange sous agitation 9,9 cm³ de 4-chloro 1-butanol et 24,3 g d'imidazole dans 50 cm³ de tétrahydrofuranne. On ajoute goutte à goutte à une température inférieure à 20°C, 2,82 g de chlorure de terbutyldiméthylsilyle, dans 20 cm³ de tétrahydrofuranne, agite 18 heures à température ambiante, essore, rince au tétrahydrofuranne et élimine le solvant sous pression réduite. On purifie le résidu par chromatographie sur silice (éluant cyclohexane-acétate d'éthyle (95-5)) et recueille 17,5 g de produit attendu.

### EXEMPLE 2 : les principes actifs suivants sont décrits et préparés comme indiqué dans les demandes de brevets européens EP-A 494819 et EP-A 0580459 :

- 3-(4-cyano 3-(trifluorométhyl) phényl 5,5-diméthyl 2,4-dioxo 1-imidazolidineacétate de (1,1-diméthyl) éthyle,
- 3-(4-cyano 3-(trifluorométhyl) phényl) 5,5-diméthyl 2,4-dioxo 1-imidazolidineacétate de cyclopentyle,
- 3-(4-cyano 3-(trifluorométhyl) phényl) 5,5-diméthyl 2,4-dioxo 1-imidazolidinebutanoate d'éthyle,
- acide 3-(4-cyano 3-(trifluorométhyl) phényl) 5,5-diméthyl 2,4-dioxo 1-imidazolidinebutanoïque,
- 3-(4-cyano 3-(trifluorométhyl) phényl) 5,5-diméthyl 2,4-dioxo 1-imidazolidinebutanoate de (1,1-diméthyl) éthyle,
- 3-(4-cyano 3-(trifluorométhyl) phényl) 5,5-diméthyl 2,4-dioxo 1-imidazolidinebutanoate de cyclopentyle,
- 4-(4,4-diméthyl 2,5-dioxo 3-(2-((4-fluorophényl) thio) éthyl) 1-imidazolidinyl 2-(trifluorométhyl) benzonitrile,
- 4-(4,4-diméthyl 2,5-dioxo 3-(2-((4-fluorophényl) sulfonyl) éthyl) 1-imidazolidinyl 2-(trifluorométhyl) benzonitrile,
- 4-(4,4-diméthyl 2,5-dioxo 3-(2-((4-fluorophényl) sulfinyl) éthyl) 1-imidazolidinyl 2-(trifluorométhyl) benzonitrile,
- 4-(4,4-diméthyl 2,5-dioxo 3-((3-méthoxyphényl) méthyl) 1-imidazolidinyl 2-(trifluorométhyl) benzonitrile,
- 4-(4,4-diméthyl 2,5-dioxo 3-(2-(4-morpholinyl) éthyl) 1-imidazolidinyl 2-(trifluorométhyl) benzonitrile,
- 4-(4,4-diméthyl 3-(2-hydroxyéthyl) 5-imino 2-thioxo 1-imidazolidinyl) 2-(trifluorométhyl) benzonitrile,
- 4-(4,4-diméthyl 3-(2-hydroxyéthyl) 5-oxo 2-thioxo 1-imidazolidinyl) 2-(trifluorométhyl) benzonitrile,
- 4-(4,4-diméthyl 3-(2-hydroxyéthyl) 5-imino 2-thioxo 1-imidazolidinyl) 2-(trifluorométhyl) 5-³H benzonitrile,
- 4-(4,4-diméthyl 3-(2-hydroxyéthyl) 5-oxo 2-thioxo 1-imidazolidinyl) 2-(trifluorométhyl) 5-³H benzonitrile,
- 4-(4,4-diméthyl 3-(3-hydroxypropyl) 5-imino 2-thioxo 1-imidazolidinyl) 2-(trifluorométhyl) benzonitrile,
- 4-(4,4-diméthyl 3-(3-hydroxypropyl) 5-oxo 2-thioxo 1-imidazolidinyl) 2-(trifluorométhyl) benzonitrile,
- 4-(4,4-diméthyl 3-(4-hydroxybutyl) 5-imino 2-thioxo 1-imidazolidinyl) 2-(trifluorométhyl) benzonitrile,
- 4-(4,4-diméthyl 3-(4-hydroxybutyl) 5-oxo 2-thioxo 1-imidazolidinyl) 2-(trifluorométhyl) benzonitrile,
- 4-(4,4-diméthyl 3-(2-méthoxyéthyl) 5-imino 2-thioxo 1-imidazolidinyl) 2-(trifluorométhyl) benzonitrile,
- 4-(4,4-diméthyl 3-(2-méthoxyéthyl) 5-oxo 2-thioxo 1-imidazolidinyl) 2-(trifluorométhyl) benzonitrile,
- 4-(4,4-diméthyl 3-(1-méthyléthyl) 5-imino 2-thioxo 1-imidazolidinyl) 2-(trifluorométhyl) benzonitrile,
- 4-(4,4-diméthyl 3-(1-méthyléthyl) 5-oxo 2-thioxo 1-imidazolidinyl) 2-(trifluorométhyl) benzonitrile,
- 3-(3,4-dichlorophényl 5,5-diméthyl 1-(3-hydroxypropyl) 4-imino 2-imidazolidine thione,
- 3-(3,4-dichlorophényl 5,5-diméthyl 1-(3-hydroxypropyl) 2-thioxo 4-imidazolidinone,
- 4-(4,4-diméthyl 3-(4-hydroxybutyl) 5-imino 2-thioxo 1-imidazolidinyl) 2-(trifluorométhyl) (5-³H) benzonitrile,
- 4-(4,4-diméthyl 3-(4-hydroxybutyl) 5-oxo 2-thioxo 1-imidazolidinyl) 2-(trifluorométhyl) (5-³H) benzonitrile,
- 4-(4,4-diméthyl 3-(4-hydroxybutyl) 5-imino 2-thioxo 1-imidazolidinyl) 2-(trifluorométhyl) benzo (¹⁴C) nitrile,
- 4-(4,4-diméthyl 3-(4-hydroxybutyl) 5-oxo 2-thioxo 1-imidazolidinyl) 2-(trifluorométhyl) benzo (¹⁴C) nitrile,
- 4-(4,4-diméthyl 3-(4-hydroxybutyl) 5-imino 2-oxo 1-imidazolidinyl) 2-(trifluorométhyl) (5-³H) benzonitrile,
- 4-(4,4-diméthyl 2,5-dioxo 3-(4-hydroxybutyl) 1-imidazolidinyl) 2-(trifluorométhyl) (5-³H) benzonitrile,
- 4-(4,4-diméthyl 3-(4-hydroxybutyl) 5-imino 2-oxo 1-imidazolidinyl) 2-(trifluorométhyl) benzo (¹⁴C) nitrile,
- 4-(4,4-diméthyl 2,5-dioxo 3-(4-hydroxybutyl) 1-imidazolidinyl) 2-(trifluorométhyl) benzo (¹⁴C) nitrile,
- 4-(2,5-dioxo 4,4-diméthyl 3-(4-triphénylméthoxybutyl) 1-imidazolidinyl) 2-(trifluorométhyl) benzonitrile,
- 4-(2,5-dioxo 4,4-diméthyl 3-(4-phénylméthoxybutyl) 1-imidazolidinyl) 2-(trifluorométhyl) benzonitrile,
- 4-[4,4-diméthyl- 2,5-dioxo 3-(4-méthoxybutyl) 1-imidazolidinyl] 2-(trifluorométhyl) benzonitrile,
- 4-[3-(4-chlorobutyl) 4,4-diméthyl 2,5-dioxo 1-imidazolidinyl] 2-(trifluorométhyl) benzonitrile,
- 4-[3-[4-[(méthylsulfonyl) oxy] butyl] 4,4-diméthyl 2,5-dioxo 1-imidazolidinyl] 2-(trifluorométhyl) benzonitrile,
- 4-(3-acétyl 4,4-diméthyl 2,5-dioxo 1-imidazolidinyl) 2-(trifluorométhyl) benzonitrile,
- 4-(3-benzoyl 4,4-diméthyl 2,5-dioxo 1-imidazolidinyl) 2-(trifluorométhyl) benzonitrile,
- 4-[3-[diméthyl (1,1-diméthyléthyl) silyl] 4,4-diméthyl 2,5-dioxo 1-imidazolidinyl] 2-(trifluorométhyl) benzonitrile,
- 1-(4-nitro-3-(trifluorométhyl) phényl)-3,4,4-triméthyl-2,5-imidazolidinedione.
- 5,5-diméthyl-1-éthyl-3-(4-nitro-3-(trifluorométhyl) phényl)-2,4-imidazolidinedione.
- 5,5-diméthyl-3-(4-nitro-3-(trifluorométhyl) phényl)-1-propyl-2,4-imidazolidine-dione.
- 5,5-diméthyl-1-(1-méthyl éthyl)-3-(4-nitro-3-(trifluorométhyl) phényl)-2,4-imidazolidinedione.
- 5,5-diméthyl-3-(4-nitro-3-trifluorométhyl) phényl)-1-(2-propényl)-2,4-imidazolidinedione.
- 5,5-diméthyl-3-(4-nitro-3-(trifluorométhyl) phényl)-1-méthyl phényl-2,4-imidazolidinedione.
- 4-(4,4-diméthyl-5-imino-2-oxo-1-imidazolidinyl)-2-(trifluorométhyl)-benzonitrile.
- 4-(4,4-diméthyl-2,5-dioxo-1-imidazolidinyl)-2-(trifluorométhyl)-benzonitrile.
- Acide 3-(4-cyano-3-(trifluorométhyl) phényl)-5,5-diméthyl-2,4-dioxo-1-imidazolidine acétique.
- 3-(4-cyano-3-(trifluorométhyl) phényl)-5,5-diméthyl-2,4-dioxo-1-imidazolidine acétate d'éthyle.
- 4-(5-imino-2-thioxo-3,4,4-triméthyl-1-imidazolidinyl)-2-(trifluorométhyl)-benzonitrile.
- 4-(5-oxo-2-thioxo-3,4,4-triméthyl-1-imidazolidinyl)-2-(trifluorométhyl)-benzonitrile.
- 4-(2,5-dithioxo-3,4,4-triméthyl-1-imidazolidinyl)-2-(trifluorométhyl)-benzonitrile.
- 4-(4,4-diméthyl-5-imino-2-thioxo-1-imidazolidinyl)-2-(trifluorométhyl)-benzonitrile.
- 4-(4,4-diméthyl-5-oxo-2-thioxo-1-imidazolidinyl)-2-(trifluorométhyl)-benzonitrile.
- 5,5-diméthyl-3-(4-nitro-3-(trifluorométhyl) phényl)-1-pentyl-2,4-imidazolidinedione.
- 5,5-diméthyl-3-(4-nitro-3-(trifluorométhyl) phényl)-1-nonyl-2,4-imidazolidinedione.
- 4-(3,4,4-triméthyl-2,5-dioxo 1-imidazolidinyl) 2-(trifluorométhyl) benzonitrile.
- 4-(5-thioxo-2-oxo-3,4,4-triméthyl-1-imidazolidinyl) 2-(trifluorométhyl)-benzonitrile (produit A) 4-(5-oxo-2-thioxo-3,4,4-triméthyl 1-imidazolidinyl) 2-(trifluorométhyl)-benzonitrile (produit B) 4-(2,5-dithioxo-3,4,4-triméthyl-1-imidazolidinyl) 2-(trifluorométhyl)-benzonitrile (produit C).
- 4-(4,5-dihydro 4,4-diméthyl 2-(méthylthio) 5-oxo 1H-imidazol-1-yl) 2-(trifluorométhyl)-benzonitrile.
- 4-[4,5-dihydro 4,4-diméthyl 5-oxo 2-[(phénylméthyl) thio] 1H-imidazol-1-yl] 2-(trifluorométhyl) benzonitrile.
- 4-[4,4-diméthyl 3-(2-hydroxyéthyl) 5-imino 2-thioxo 1-imidazolidinyl] 2-(trifluorométhyl) benzonitrile.
- 4-[4,4-diméthyl 3-(2-hydroxyéthyl) 5-oxo 2-thioxo 1-imidazolidinyl] 2-(trifluorométhyl) benzonitrile (Produit A) et 4-[4,4-diméthyl 2,5-dioxo 3-(2-mercaptoéthyl) 1-imidazolidinyl] 2-(trifluorométhyl) benzonitrile (Produit B).
- 4-(4,4-diméthyl 2,5-dioxo 3-éthyl 1-imidazolidinyl) 2-(trifluorométhyl) benzonitrile.
- 4-(4,4-diméthyl 2,5-dioxo 3-(2-propényl) 1-imidazolidinyl) 2-(trifluorométhyl) benzonitrile.
- 4-(4,4-diméthyl 2,5-dioxo 3-(phénylméthyl) 1-imidazolidinyl) 2-(trifluorométhyl) benzonitrile.
- 4-[4,4-diméthyl 2,5-dioxo 3-[(4-fluorophényl) méthyl] 1-imidazolidinyl] 2-(trifluorométhyl) benzonitrile.
- 4-[4,4-diméthyl 2,5-dioxo 3-[(4-méthoxyphényl) méthyl] 1-imidazolidinyl] 2-(trifluorométhyl) benzonitrile.
- 4-[4,4-diméthyl 2,5-dioxo 3-[[4-[trifluorométhyl) phényl] méthyl] 1-imidazolidinyl] 2-(trifluorométhyl) benzonitrile.
- 4-[4,4-diméthyl 2,5-dioxo 3-(2-époxyméthyl) 1-imidazolidinyl] 2-(trifluorométhyl) benzonitrile.
- 4-(4,4-diméthyl 2,5-dioxo 3-propyl-1H-imidazolidinyl) 2-(trifluorométhyl) benzonitrile.
- 4-(4,4-diméthyl 2,5-dioxo 3-(1-méthyléthyl) 1-imidazolidinyl] 2-(trifluorométhyl) benzonitrile.
- 4-[4,5-dihydro 4,4-diméthyl 2-(nonylthio) 5-oxo 1H-imidazol-1-yl] 2-(trifluorométhyl) benzonitrile.
- 4-[4,5-dihydro 4,4-diméthyl 2-[(3-hydroxypropyl) thio] 5-oxo 1H-imidazol-1-yl] 2-(trifluorométhyl) benzonitrile.
- [[1-[4-cyano 3-(trifluorométhyl) phényl] 4,5-dihydro 4,4-diméthyl 5-oxo 1H-imidazol-2-yl] thio] acétate d'éthyle.
- 4-(4,4-diméthyl 3-éthyl 5-imino 2-thioxo 1-imidazolidinyl) 2-(trifluorométhyl) benzonitrile.
- 4-(4,4-diméthyl 5-imino 3-pentyl 2-thioxo 1-imidazolidinyl) 2-(trifluorométhyl) benzonitrile.
- 4-(4,4-diméthyl 3-éthyl 5-oxo 2-thioxo 1-imidazolidinyl) 2-(trifluorométhyl) benzonitrile.
- 4-(4,4-diméthyl 5-oxo 3-pentyl 2-thioxo 1-imidazolidinyl) 2-(trifluorométhyl) benzonitrile.
- 4-[4,5-dihydro 4,4-diméthyl 2-(méthylthio) 5-thioxo 1H-imidazol-1-yl] 2-(trifluorométhyl) benzonitrile.
- 4-[4,5-dihydro 4,4-diméthyl 2-[(phénylméthyl) thio] 5-thioxo 1H-imidazol-1-yl] 2-(trifluorométhyl) benzonitrile.
- 3-[4-cyano 3-(trifluorométhyl) phényl] 5,5-diméthyl 2,4-dioxo N-méthyl N-(1-méthyléthyl) 1-imidazolidine acétamide.
- 4,-[4,4-diméthyl 2,5-dioxo 3-(2-hydroxyéthyl) 1-imidazolidinyl] 2-(trifluorométhyl) benzonitrile.
- 4-[4,4-diméthyl 2,5-dioxo 3-(3-hydroxypropyl) 1-imidazolidinyl] 2-(trifluorométhyl) benzonitrile.
- 4-[3-[2-(acétyloxy) éthyl] 4,4-diméthyl 2,5-dioxo 1-imidazolidinyl] 2-(trifluorométhyl) benzonitrile.
- 4-[4,4-diméthyl 2,5-dioxo 3-(5-hydroxypentyl) 1-imidazolidinyl] 2-(trifluorométhyl) benzonitrile.
- 4-[4,4-diméthyl 2,5-dioxo 3-(2-méthoxyéthyl) 1-imidazolidinyl] 2-(trifluorométhyl) benzonitrile.
- 4-[4,4-diméthyl 2,5-dioxo 3-(cyanométhyl) 1-imidazolidinyl] 2-(trifluorométhyl) benzonitrile.
- 4-[4,4-diméthyl 2,5-dioxo 3-[(1,3-dioxalan-2-yl) méthyl] 1-imidazolidinyl] 2-(trifluorométhyl) benzonitrile.
- 4-[4,4-diméthyl 2,5-dioxo 3-(2-chloroéthyl) 1-imidazolidinyl] 2-(trifluorométhyl) benzonitrile.
- 1-(3,4-dichlorophényl) 5-imino 3,4,4-triméthyl 2-imidazolidine thione.
- 3-(3,4-dichlorophényl) 2-thioxo 1,5,5-triméthyl 4-imidazolidinone.
- 3-(3,4-dichlorophényl) 3,5-dihydro 5,5-diméthyl 2-(méthylthio) 4H-imidazol-4-one.
- 1-(3,4-dichlorophényl) 3,4,4-triméthyl 2,5-imidazolidine dithione.
- 1-[4-chloro 3-(trifluorométhyl) phényl] 4,4-diméthyl 2-thioxo 5-imidazolidinone.
- 1-[4-chloro 3-(trifluorométhyl) phényl] 4,4-diméthyl 5-imino 2-imidazolidine thione.
- 3-(3,4-dichlorophényl) 3,5-dihydro 5,5-diméthyl 2-[(phénylméthyl) thio] 4H-imidazol-4-one.

### EXEMPLE 3 : Procédé de préparation d'une composition comprenant des liposomes

On ajoute 0,13 g d'α-tocophérol à un mélange comprenant 0,5 g du principe actif 4-(4,4-diméthyl 2,5-dioxo 3-(4-hydroxybutyl) 1-imidazolidinyl) 2-(trifluorométhyl) benzonitrile et 10,77 g de phospholipide (lipoid E.100.35).

Le mélange est chauffé en agitant à une température supérieure à la température de transition du phospholipide, préférablement à 60°C-70°C. La température de transition du phospholipide choisi est préférentiellement environ 60°C.

On prépare une solution qui contient : 0,1 g de para-hydroxybenzoate de méthyle et 0,05 g de para-hydroxybenzoate de propyle dans 100 ml de solution de tampon pH 7 en chauffant à une température élevée (i.e. 100°C).

Cette solution est ajoutée au mélange du principe actif et de phospholipide.

On ajoute de l'acétate d'α-tocophérol ou de l'α-tocophérol à cette solution. On agite jusqu'à ce que le mélange soit homogène.

Cette solution est passée dans un microfluidiseur (1 à 6 passages, pression 15 à 140 MPa). La préparation peut être utilisée telle quelle ou on peut y incorporer un gélifiant comme l'hydroxypropyl cellulose (Blanose T6F) à raison de 1 % ou tout autre gélifiant.

La composition finale contient des liposomes dont la taille est comprise entre 40 et 120 nm.

La composition peut être utilisée pour le traitement des maladies et dans la cosmétologie ou dermatologie.

### EXEMPLE 4 :

Par analogie avec la préparation décrite à l'exemple 3, on prépare des compositions comprenant des liposomes qui contiennent les composés actifs de l'exemple 2.

## Revendications

1. Procédé de préparation de compositions cosmétiques ou pharmaceutiques comprenant des liposomes qui contiennent un composé actif, **caractérisées en ce que** les liposomes contiennent comme principe actif au moins un composé de formule (I) : 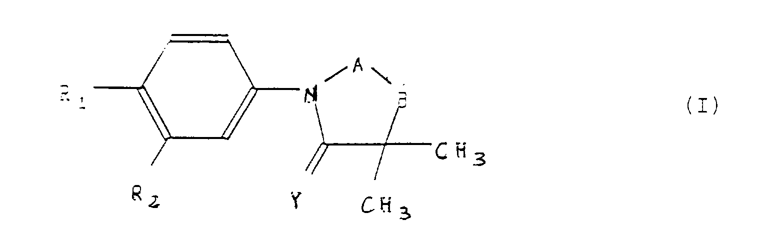 dans laquelle :
R₁ représente un radical cyano ou nitro ou un atome d'halogène,
R₂ représente un radical trifluorométhyle ou un atome d'halogène,
le groupement -A-B- est choisi parmi les radicaux 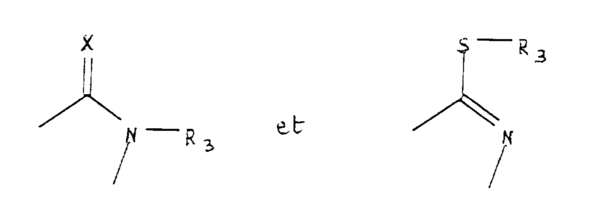 dans lesquels X représente un atome d'oxygène ou de soufre et
R₃ est choisi parmi les radicaux suivants :
- un atome d'hydrogène,
- les radicaux alkyle, alkényle, alkynyle, aryle ou arylalkyle ayant au plus 12 atomes de carbone, ces radicaux étant éventuellement substitués par un ou plusieurs substituants choisis parmi les radicaux hydroxy, halogène, mercapto, cyano, acyle ou acyloxy ayant au plus 7 atomes de carbone, aryle, O-aryle, O-aralkyle, S-aryle dans lesquels le radical aryle renfermant au plus 12 atomes de carbone est éventuellement substitué et l'atome de soufre est éventuellement oxydé sous forme de sulfoxyde ou de sulfone, carboxy libre, estérifié, amidifié ou salifié, amino, mono ou dialkylamino ou un radical hétérocyclique comprenant 3 à 6 chaînons et renfermant un ou plusieurs hétéroatomes choisis parmi les atomes de soufre, d'oxygène ou d'azote,
les radicaux alkyle, alkényle ou alkynyle étant de plus éventuellement interrompus par un ou plusieurs atomes d'oxygène, d'azote ou de soufre éventuellement oxydé sous forme de sulfoxyde ou de sulfone,
les radicaux aryle et aralkyle étant de plus éventuellement substitués par un atome d'halogène, par un radical alkyle, alkényle ou alkynyle, alkoxy, alkényloxy, alkynyloxy ou trifluorométhyle,
- les radicaux trialkylsilyle dans lesquels le radical alkyle linéaire ou ramifié comprend au plus 6 atomes de carbone,
- les radicaux acyle ou acyloxy renfermant au plus 7 atomes de carbone,
Y représente un atome d'oxygène ou de soufre ou un radical =NH, ce procédé étant **caractérisé en que** l'on prépare une émulsion ou suspension aqueuse comprenant un composé actif et un ou plusieurs composés lipidiques et éventuellement des additifs comme un tampon de pH, un antioxydant ou un antiseptique et que l'on agite l'émulsion ou la suspension à une température comprise entre 40° et 80°C et que l'on applique ensuite un procédé de réduction de taille des liposomes.

2. Procédé de préparation selon la revendication 1, **caractérisé en ce que** l'on utilise comme composé actif un composé de formule (I) telle que définie dans la revendication 1 dans lequel X représente un atome d'oxygène, Y représente un atome d'oxygène, R₂ représente un atome d'halogène ou un radical trifluorométhyle, R₁ représente un radical nitro, un atome d'halogène ou un radical cyano et R₃ représente un atome d'hydrogène ou un radical alkyle ayant au plus 4 atomes de carbone éventuellement substitué par un radical hydroxy ou méthoxy.

3. Procédé de préparation selon la revendication 1, **caractérisé en ce que** l'on utilise comme composé actif un composé de formule (I') :

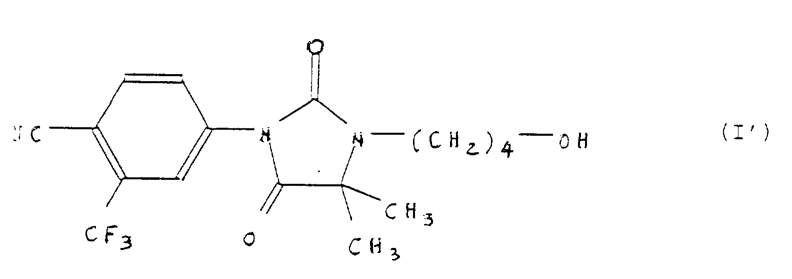

4. Procédé de préparation selon l'une quelconque des revendications 1 à 3, **caractérisé en ce que** l'on utilise comme composé lipidique un phospholipide.

5. Procédé de préparation selon l'une quelconque des revendications 1 à 3, **caractérisé en ce que** l'on utilise comme composé lipidique le phospholipide E 100.35, EPC 3 ou SPC 3.

6. Procédé de préparation selon l'une quelconque des revendications 1 à 3, **caractérisé en ce que** l'on agite l'émulsion ou la suspension à une température comprise entre 40° et 75°C et que l'on applique ensuite comme procédé de réduction de taille des liposomes un ou plusieurs passages de l'émulsion ou de la suspension dans un microfluidiseur.

## Patentansprüche

1. Verfahren zur Herstellung von kosmetischen oder pharmazeutischen Zusammensetzungen, die Liposomen umfassen, die einen Wirkstoff enthalten, **dadurch gekennzeichnet, dass** die Liposomen als Wirkstoff wenigstens eine Verbindung der Formel (I): 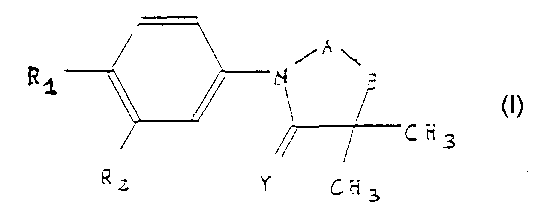 enthalten, in der:
R₁ einen Cyano- oder Nitrorest oder ein Halogenatom darstellt,
R₂ einen Trifluormethylrest oder ein Halogenatom darstellt, die -A-B-Gruppe unter den Resten 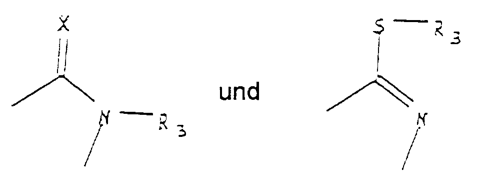 ausgewählt ist, in denen X ein Sauerstoff- oder Schwefelatom darstellt und R₃ unter den folgenden Resten ausgewählt ist:
- einem Wasserstoffatom,
- den Resten Alkyl, Alkenyl, Alkinyl, Aryl oder Arylalkyl mit höchstens 12 Kohlenstoffatomen, wobei diese Reste gegebenenfalls mit einem oder mehreren Substituenten substituiert sind, die ausgewählt sind unter den Resten Hydroxy, Halogen, Mercapto, Cyano, Acyl oder Acyloxy mit höchstens 7 Kohlenstoffatomen, Aryl, O-Aryl, O-Aralkyl, S-Aryl, in denen der Arylrest mit höchstens 12 Kohlenstoffatomen gegebenenfalls substituiert ist und das Schwefelatom gegebenenfalls in Form von Sulfoxid oder Sulfon oxidiert ist, Carboxy in freier, Ester-, Amid- oder Salzform, Amino, Mono- oder Dialkylamino oder einem heterocyclischen Rest mit 3 bis 6 Ringgliedern, der ein oder mehrere Heteroatome, die unter den Schwefel-, Sauerstoff oder Stickstoffatomen ausgewählt sind, umfasst,
den Resten Alkyl, Alkenyl oder Alkinyl, die außerdem gegebenenfalls durch ein oder mehrere Sauerstoff-, Stickstoff- oder Schwefelatome, gegebenenfalls in Form von Sulfoxid oder Sulfon oxidiert, unterbrochen sind,
den Resten Aryl und Aralkyl, die außerdem gegebenenfalls mit einem Halogenatom, mit einem Rest Alkyl, Alkenyl oder Alkinyl, Alkoxy, Alkenyloxy, Alkinyloxy oder Trifluormethyl substituiert sind,
- den Trialkylsilylresten, in denen der lineare oder verzweigte Alkylrest höchstens 6 Kohlenstoffatome umfasst,
- den Acyl- oder Acyloxyresten mit höchstens 7 Kohlenstoffatomen,
Y ein Sauerstoff- oder Schwefelatom oder einen =NH-Rest darstellt, wobei dieses Verfahren **dadurch gekennzeichnet ist, dass** man eine wässrige Emulsion oder Suspension herstellt, die einen Wirkstoff und eine oder mehrere Lipidverbindungen und gegebenenfalls Zusatzstoffe, wie einen pH-Puffer, ein Antioxidationsmittel oder ein Antiseptikum umfasst, und dass man die Emulsion oder die Suspension bei einer Temperatur zwischen 40 ° und 80 °C rührt und dass man dann ein Verfahren zur Verminderung der Größe der Liposomen anwendet.

2. Verfahren zur Herstellung gemäß Anspruch 1, **dadurch gekennzeichnet, dass** man als Wirkstoff eine Verbindung der Formel (I), wie in Anspruch 1 definiert, verwendet, in der X ein Sauerstoffatom darstellt, Y ein Sauerstoffatom darstellt, R₂ ein Halogenatom oder einen Trifluormethylrest darstellt, R₁ einen Nitrorest, ein Halogenatom oder einen Cyanorest darstellt und R₃ ein Wasserstoffatom oder einen Alkylrest mit höchstens 4 Kohlenstoffatomen, der gegebenenfalls mit einem Hydroxy- oder Methoxyrest substituiert ist, darstellt.

3. Verfahren zur Herstellung gemäß Anspruch 1, **dadurch gekennzeichnet, dass** man als Wirkstoff eine Verbindung der Formel (I'): 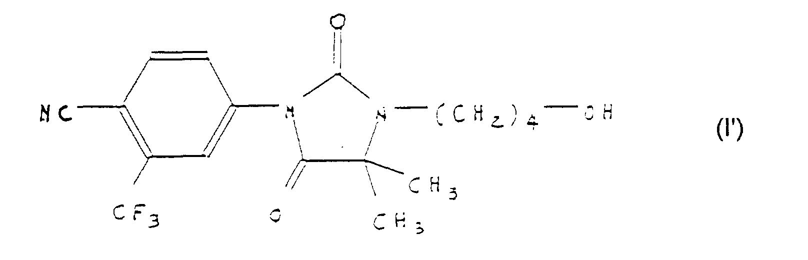 verwendet.

4. Verfahren zur Herstellung gemäß einem der Ansprüche 1 bis 3, **dadurch gekennzeichnet, dass** man als Lipidverbindung ein Phospholipid verwendet.

5. Verfahren zur Herstellung gemäß einem der Ansprüche 1 bis 3, **dadurch gekennzeichnet, dass** man als Lipidverbindung das Phospholipid E 100.35, EPC 3 oder SPC 3 verwendet.

6. Verfahren zur Herstellung gemäß einem der Ansprüche 1 bis 3, **dadurch gekennzeichnet, dass** man die Emulsion oder die Suspension bei einer Temperatur zwischen 40 ° und 75 °C rührt und dass man dann als Verfahren zur Verminderung der Größe der Liposomen ein oder mehrere Durchgänge der Emulsion oder der Suspension durch einen Mikrofluidisierer anwendet.

## Claims

1. Preparation process for cosmetic or pharmaceutical compositions comprising liposomes which contain an active compound, **characterised in that** the liposomes contain as an active ingredient at least one compound of formula (I): 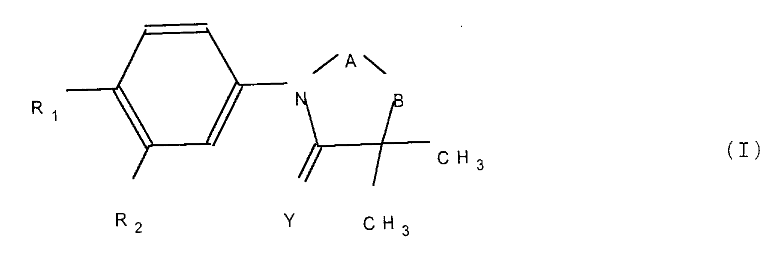 in which:
R₁ represents a cyano or nitro radical or a halogen atom,
R₂ represents a trifluoromethyl radical or a halogen atom, the -A-B- group is chosen from the 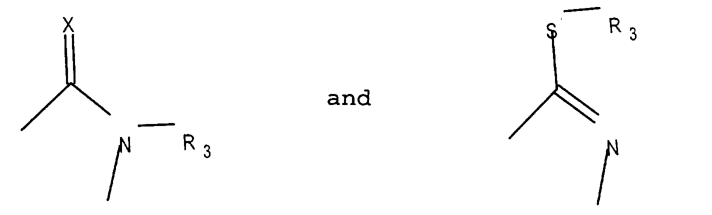 radicals in which X represents an oxygen or sulphur atom and
R₃ is chosen from the following radicals:
- a hydrogen atom,
- the alkyl, alkenyl, alkynyl, aryl or arylalkyl radicals having at most 12 carbon atoms, these radicals being optionally substituted by one or more substituents chosen from the hydroxy, halogen, mercapto, cyano, acyl or acyloxy having at most 7 carbon atoms, aryl, O-aryl, O-aralkyl, S-aryl radicals in which the aryl radical containing at most 12 carbon atoms is optionally substituted and the sulphur atom is optionally oxidized in the form of sulphoxide or sulphone, free, esterified, amidified or salified carboxy, amino, mono or dialkylamino or a heterocyclic radical comprising 3 to 6 members and containing one or more heteroatoms chosen from the sulphur, oxygen or nitrogen atoms, the alkyl, alkenyl or alkynyl radicals in addition being optionally interrupted by one or more oxygen, nitrogen or sulphur atoms optionally oxidized in the form of sulphoxide or sulphone,
the aryl and aralkyl radicals in addition being optionally substituted by a halogen atom, by an alkyl, alkenyl or alkynyl, alkoxy, alkenyloxy, alkynyloxy or trifluoromethyl radical,
- the trialkylsilyl radicals in which the linear or branched alkyl radical contains at most 6 carbon atoms,
- the acyl or acyloxy radicals containing at most 7 carbon atoms,
Y represents an oxygen or sulphur atom or an =NH radical, this process being **characterized in that** an aqueous emulsion or suspension containing an active compound and one or more lipidic compounds and optionally additives such as a pH buffer, an antioxidant or an antiseptic is prepared and that the emulsion or the suspension is agitated at a temperature of between 40° and 80°C and that a process of reducing the size of the liposomes is then applied.

2. Preparation process according to claim 1, **characterized in that** a compound of formula (I) as defined in claim 1 in which X represents an oxygen atom, Y represents an oxygen atom, R₂ represents a halogen atom or a trifluoromethyl radical, R₁ represents a nitro radical, a halogen atom or a cyano radical and R₃ represents a hydrogen atom or an alkyl radical having at most 4 carbon atoms optionally substituted by a hydroxy or methoxy radical is used as an active ingredient.

3. Preparation process according to claim 1, **characterized in that** a compound of formula (I') is used as an active ingredient:

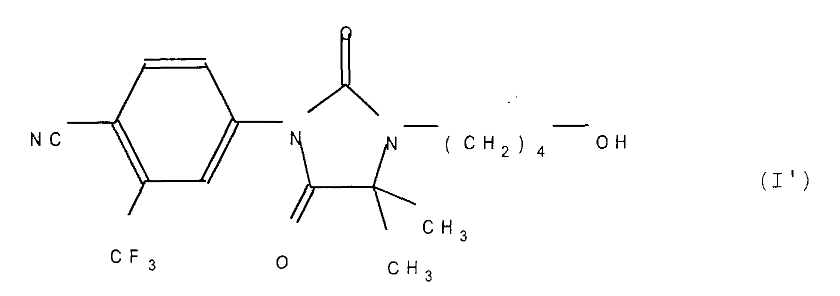

4. Preparation process according to any one of claims 1 to 3, **characterized in that** a phospholipid is used as lipidic compound.

5. Preparation process according to any one of claims 1 to 3, **characterized in that** the phospholipid E 100.35, EPC 3 or SPC 3 is used as lipidic compound.

6. Preparation process according to any one of claims 1 to 3, **characterized in that** the emulsion or the suspension is agitated at a temperature comprised between 40° and 75°C and that one or more passes of the emulsion or the suspension through a microfluidizer are then carried out as a process to reduce the size of the liposomes.
